# EUROPEAN PATENT SPECIFICATION

(11) **EP 1 736 112 B1**
(45) Date of publication and mention of the grant of the patent: **17.08.2011**
(21) Application number: 05013240.6
(22) Date of filing: 20.06.2005
(51) Int. Cl.: A61C 1/00, A61C 3/04

(54) **Medical device**
Medizinische Vorrichtung
Dispositif médical

(43) Date of publication of application: 27.12.2006
(73) Proprietor: Schmid, Heribert, 82194 Gröbenzell (DE)
(72) Inventor: Schmid, Heribert, 82194 Gröbenzell (DE); Unrecht, Gerhard, 82110 Germering (DE); Schmidt, Jörg, 82272 Moorenweis (DE)
(74) Representative: TBK

(56) References cited:
- EP-A- 1 155 654
- WO-A-2004/019893
- US-A1- 2002 193 709
- US-A1- 2003 116 159

## Description

The present invention relates to a medical device according to the preamble of claim 1 which comprises a medical instrument such as a drill and data exchange means. The medical instrument is preferably formed as a dental drill and comprises additionally a storage medium member or in a preferred form a transponder which serves as a data medium about specific information of the medical instrument. The specific information of the medical instrument are exchanged between the transponder of the medical instrument and the data exchange means.

Furthermore, the present disclosure relates to a magazine for receiving at least one medical instrument of the medical device which is preferably in the form of the above-mentioned dental drill with a transponder fixed thereon.

In general, so called dental drills known as medical instruments are used for the dental treatment. These dental drills are repeatedly used whereby the material they are made of suffers from material fatigue. This considering as example causes the dental drills to break during a dental treatment which corresponds to the worst case or the result of the dental treatment by the dental drill is not satisfying due to the wear of the material of the dental drill or due to the material fatigue.

In order to prevent such above-mentioned cases the state of the medical instrument can be identified and monitored continuously. For example material properties can be monitored of the corresponding medical instrument and can be updated after every medical treatment. Consequently, the state of the medical instrument is monitored and identified which can be done by evaluating certain values specific for the corresponding medical instrument and the material thereof, so that the medical instrument can be replaced by a new one if the evaluation of the values leads to the result that the risk of breaking or the wear of the medical instrument is high.

Values for evaluating the state of the medical instrument could for example be specific information on the use of the medical instrument which is in more detail one or more of the position of the medical instrument in a magazine, the specific accumulated total load depending on the medical instrument, the accumulated partial load depending on the medical instrument and the number of sterilisation cycles carried out. Furthermore, the specific information on the use of the medical instrument can additionally comprise parameters like one or more of the type, the structure of the medical instrument and parameters referring to the medical instrument itself, especially one or more of the torque, the rotational direction and the rotational number of the medical instrument in the case of a rotational instrument like a dental drill.

Such a device is already known by the prior art which makes use of such a technical teaching. In view of the present invention, the prior art document WO2004/019803 discloses a medical device quite similar.

That prior art medical device for dental treatment consists substantially of two parts which are one angled part and one holding part. These two parts are detachably mounted to each other. The angled part includes a medical instrument mounting head for mounting the medical instrument thereto. Furthermore, a data exchange means is disposed between the angled part and the holding part. For example, the data exchange means can comprise induction coils each of which are located at the angled part and the holding part, respectively. The medical instrument which is in the preferred embodiment a dental drill has a transponder formed thereon which establishes a connection with the data exchange means on the side of the angled part, namely the induction coils on the side of the angled part. In more detail, a reading/writing means is formed in the medical instrument mounting head which is in connection with the data exchange means on the side of the angled part. This reading/writing means is in contact with the transponder formed on the medical instrument in order to read and write data from or to the transponder referring to specific information of the medical instrument mentioned above.

Since the transponder is formed on the medical instrument fixedly which is inserted in the medical instrument mounting head, the transponder is exposed to the same influences caused by the dental treatment as the medical instrument is exposed to.

In the case of a rotating medical instrument, a further load on the transponder is acting. While the transponder which is formed on the medical instrument fixedly is rotated with the medical instrument, the connection between the reading/writing means and the transponder has to be maintained. This can be achieved by a continuous physical contact between the transponder and the reading/writing means, so that the transponder wears for example.

Even other possibilities for maintaining the connection between the above-mentioned both members lead to high cost solutions which are not appropriate for a medical instrument which is replaced by a newer one if it wears out.

For that reasons, the invention has the object of improving such a prior art device, especially of preventing or reducing disadvantageous influences caused by the medical treatment for the transponder.

This object is solved by the medical device according to claim 1. Especially, the above-mentioned object is solved in that the medical instrument is placed to receiving means for receiving the medical instrument which might be for example the medical instrument mounting head and is separated from the transponder, while the transponder is placed to a data exchange means which is locally placed apart or locally placed different from the receiving means.

Thus, the transponder is not exposed to the influences depending on the medical treatment and the treatment vicinity of the medical instrument. Therefore the transponder is not disadvantageously influenced.

Furthermore, the connection between the transponder and the data exchange means does not have to be complicated or resistant against any kind of environmental influences. Accordingly, the costs for the connections for example between the transponder and the data exchange means or the reading/writing means can be held cheap.

A further advantage to the prior art is that, in the case of a rotating medical instrument, the contact portions of the transponder and the data exchange means do not wear compared to the prior art, since the transponder is spaced or placed apart from the medical instrument in the present invention and is not a rotated member. Therefore the transponder is not exposed to any environmental influences and its durability is improved.

Further advantageous effects of the present invention are subject-matter of the dependent claims.

### BRIEF DESCRIPTION OF THE DRAWINGS

The present invention can better be understood in connection with the detailed description of the preferred embodiment together with the accompanying drawings which are as follows:
Fig. 1 is a perspective view of the medical device of the present invention.
Fig. 2 is a perspective view of the transponder of the medical instrument which is in the form of a sleeve according to the present invention.
Fig. 3 is a perspective view of the magazine for storing a plurality of medical instruments.
Fig. 4 is a flow chart illustrating a procedure to be performed by an electronic control unit when using the medical device.

### DETAILED DESCRIPTION OF THE PREFERRED EMBODIMENTS

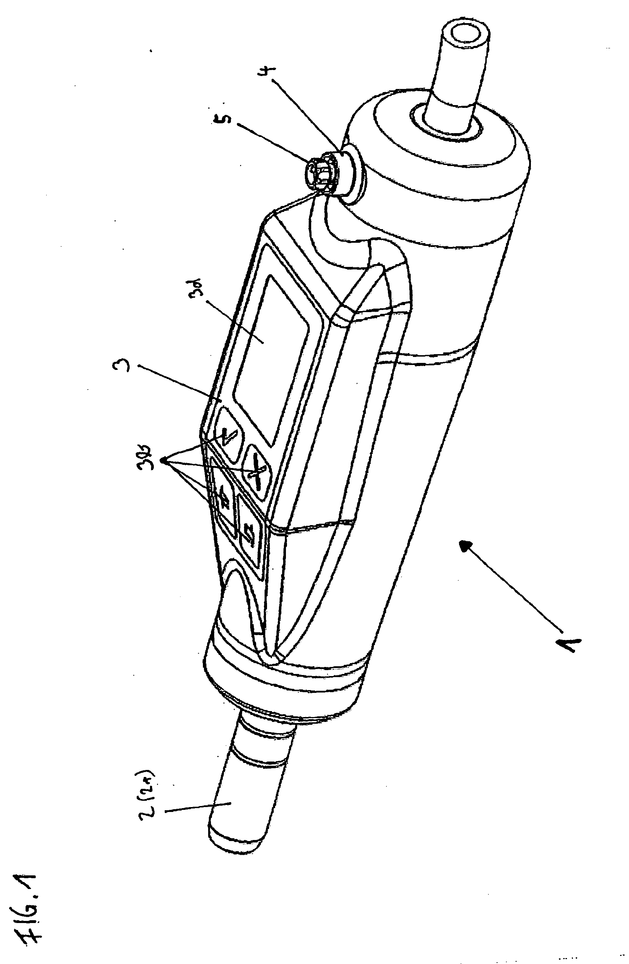

A medical device 1 according to the present invention is shown in figure 1 which is a perspective view thereof. In that specific case a medical device 1 for exemplary dental treatment is represented but the invention may also be applied to other medical devices 1 used in other technical or medical fields. This medical device 1 may be a conventional medical device 1 known by the prior art comprising the corresponding means for the respective medical treatment which are incorporated herein by reference to the prior art document mentioned in the introductory part of this specification.

Especially, the medical device 1 comprises (among other things as mentioned above) a receiving means 2 for receiving a medical instrument 2a, an operating means 3 for operating the medical device 1 and a data exchange means 4 for exchanging data with a storage medium member, preferably a transponder 5 formed detachably on the medical instrument 2a which will be described later in more detail. These specific three means 2, 3 and 4 are preferably arranged in a certain sequence so that the medical device 1 could be divided into portions, namely a medical treatment portion with the receiving means 2 which is next to an operating portion with the operating means 3. The operating portion is disposed next to a data exchange portion with the data exchange means 4. Thus, the medical treatment portion and the data exchange portion are spaced or placed apart from each other. However, that arrangement is not obligatory as long as the receiving means 2 and the data exchange means 4 are space apart from each other so that the data exchange means 4 is not affected by influences caused by the medical treatment.

The receiving means 2 for receiving the medical instrument 2a could exemplary be in the form of medical instrument mounting head to which the medical instrument 2a is mounted in a fixed manner. Especially, this medical instrument mounting head could have the shape of an angled piece or an elbow which is detachably mounted to the medical device 1. To one end of that angled piece the medical instrument 2a is mounted, while the other end of that angled piece is detachably mounted to the medical device 1.

The type of fixation between the medical instrument 2a and the receiving means 2 depends on the type of receiving means 2 which can be for example a rotational receiving means 2r or a non-rotational receiving means 2n.

In the preferred case of a rotational receiving means 2r, the medical instrument 2a detachably mounted thereto is formed as a rotating member which is in the preferred embodiment a dental drill 2d. That dental drill 2d could be fixed to the rotational receiving means 2r by exemplary a gripping mechanism (not shown). However, other mechanisms for fixing the dental drill 2d to the rotational receiving means 2r can also be used which are for example a form fitting mechanism or a combination of a form fitting mechanism and a gripping mechanism and the like.

In the case of a non-rotational receiving means 2n, fixations known by the prior art can be used which are for example a clamping connection, an engaging connection, a click-into-connection and the like.

That is, each type of fixation can be used, irrespective of the rotational receiving means 2r or the non-rotational receiving means 2n, as long as the corresponding fixation is suitable for the respective medical treatment.

Furthermore, the medical device 1 comprises an operating means 3 for operating the medical device 1 according to the intended activity to be performed. This could be for example the adjustment of the rotational speed in the case of the rotational receiving means 2r and other activities to be performed for the corresponding medical treatment. The operating means 3 can be in the shape of an operating surface formed on the medical device 1 comprising for example an operating display 3d for illustrating exemplary the state of the medical device 1 and a plurality of operating buttons 3b for controlling the corresponding operations of the medical device 1 like for example controlling the rotational speed of the medical instrument 2a in the case of the rotational receiving means 2r. Therefore, the operating buttons function as controlling means for controlling the operations of the medical device 1.
However, any other type of operating means 3 can be used. For example the operating means 3 with the controlling means 3b does not necessarily have to be formed on the medical device 1. There can also be an operating means 3s of a different kind which is separated from the medical device 1 and connected therewith via a radio communication or a cable connection.

As mentioned above the medical device 1 further comprises a data exchange means 4 for exchanging data with a storage medium member, preferably a transponder 5 mounted detachably to the medical instrument 2a which will be described in more detail later as mentioned-above.

The data exchange means 4 is disposed apart from the receiving means 2 or placed locally different from the receiving means 2 and comprises an engaging element 4e and a data exchanging element 4d, wherein the engaging element 4e serves for the corresponding engagement with the storage medium member, preferably the transponder 5 of the medical instrument 2a. Thus, the transponder 5 also includes an engaging means 5e to establish the engagement between the transponder 5, i.e. the engaging means 5e and the engaging element 4e of the data exchange means 4.

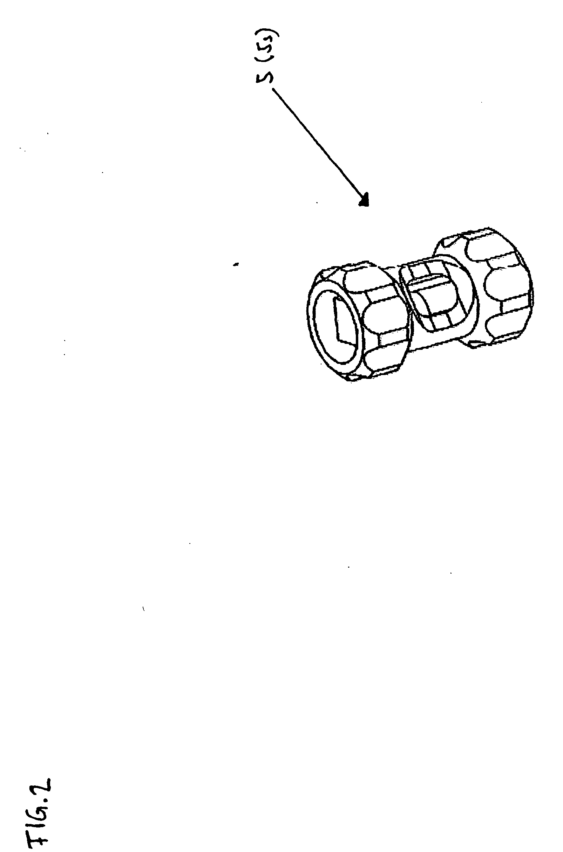

The engaging element 4e of the data exchange means 4 is preferably formed as pedestal shaped element protruding from a surface of the medical device 1. Furthermore, the engaging element 4e is preferred to be in a hollow cylindrical shape. In the case of the hollow cylindrical shaped engaging element 4h, the transponder 5 needs to have the same shape like the engaging element 4h to be inserted in the hollow cylindrical shaped engaging element 4h. Therefore the transponder 5 has to be in the shape of exemplary a sleeve 5s (shown in Fig. 2) which has a smaller diameter than the hollow cylindrical shaped engaging element 4h.

The engagement between the transponder 5 or the engaging means 5e of the transponder 5 and the engaging element 4e can be established by an engagement already known by the prior art. Preferred engagement connections are for example a frictionally engaged connection or a form fitted connection which can especially be a plug-and-socket-connection, a click-into-connection or a clamp-connection. But other connections or types of engagements are also considerable as long as a suitable and sufficient engagement between the engagement element 4e of the data exchange means 4 and the transponder 5 can be established.

Furthermore, the data exchange means 4 also provides a data exchanging element 4d which establishes, besides the (mechanical) connection or engagement between the transponder 5 and the engaging element 4e, a connection able to exchange data or specific information between both above-mentioned elements. That is, this element is functioning as a reading/writing means which is able to read data from the transponder 5 and writing data to the transponder 5. Furthermore, that element is able to process the read-in data and evaluate them whereby this element is also able to function as a calculating means and may be formed as an electronic control unit for example.

The type of data exchanging connections can be any known by the prior art which are incorporated herein by reference to the prior art document mentioned in the introductory part of the specification.

For example, the transponder 5 includes a connector (not shown) which is connected with an corresponding connector of the data exchanging means 4. In this way data exchange is performed but, however, other types of data exchanging mechanisms can be used, for example by means of inductive coils and the like.

Consequently, the transponder 5 and the medical instrument 2a which are first of all mounted detachably together are separated from each other before using the medical device 1 which will be described later. While the medical instrument 2a is fitted to the receiving means 2 which is in the preferred embodiment a rotational receiving means 2r representing a medical instrument mounting head, the transponder 5 is inserted in the data exchange means 4 so that the influence of the medical treatment or the operations carried out with the medical instrument 2a does not affect the transponder 5.

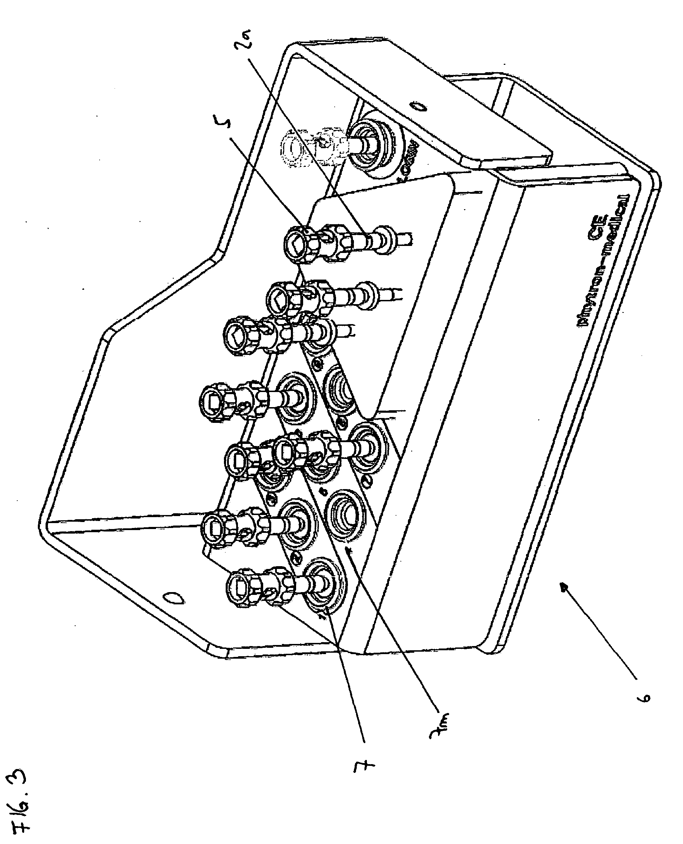

Hereinafter the storage which is designated as magazine 6 in the following (shown in Fig. 3) where the medical instrument(s) 2a are stored before being installed on the medical device 1 will be explained. It is noted that the magazine 6 may have a part or all of the features of the magazine already known from the prior art document mentioned in the introductory part of the specification.

The magazine 6 has preferably the shape of a box which has on one surface (which is the upper surface in this embodiment) one or more storage portion(s) formed therein which is in the preferred form a hole 7 in this preferred embodiment. These holes 7 serve as storing portions for the medical instruments 2a. However, these storage portions may have each shape as long as they are suitable to store or hold the medical instrument 2a.

It has to be noted that if a medical instrument 2a is inserted into one hole 7, the transponder 5 is detachably mounted onto the medical instrument 2a. The type of fixation between the transponder 5 and the medical instrument 2a may be one of the above-mentioned types as long as the transponder 5 is mounted onto the medical instrument 2a detachably. Thus, the respective transponder 5 having saved the respective specific information of the corresponding medical instrument 2a is mounted onto the respective medical instrument 2a so that no confusion may occur between any transponder 5 and the medical instrument 2a belonging to the respective transponder 5.

The holes 7 may be identified with markings so that it is made sure that the respective medical instrument 2a is inserted into the correct hole 7. Also the magazine may contain any means similar to the above-mentioned means of medical device 1. Insofar the magazine could also be used to determine the state or condition of a respective medical instrument 2a or have the function of monitoring and identifying the respective medical instrument.
As mentioned above the magazine may include all the features known from the prior art and therefore may be embodied in various forms.

Additionally, the magazine 6 may be provided with a storing portion for a box or magazine specific transponder 5s. This box specific transponder 5s may contain information (among other things) about the arrangement of the medical instruments 2a detachably mounted to the corresponding storing portions, information about the medical treatment to be carried out and, accordingly, for example the sequence in which the medical instruments 2a have to be used during the corresponding medical treatment. Consequently, each magazine 6 which is provided with such a storing portion for a box specific transponder 5s can be distinguished by the latter so that always the correct magazine having the respective medical instruments suitable for the specific medical treatment is used. The information stored in the box specific transponder 5s can be read out by for example the data exchange means 4 of the medical device 1 itself or any kind of device having a data exchange means suitable for the box specific transponder 5s.

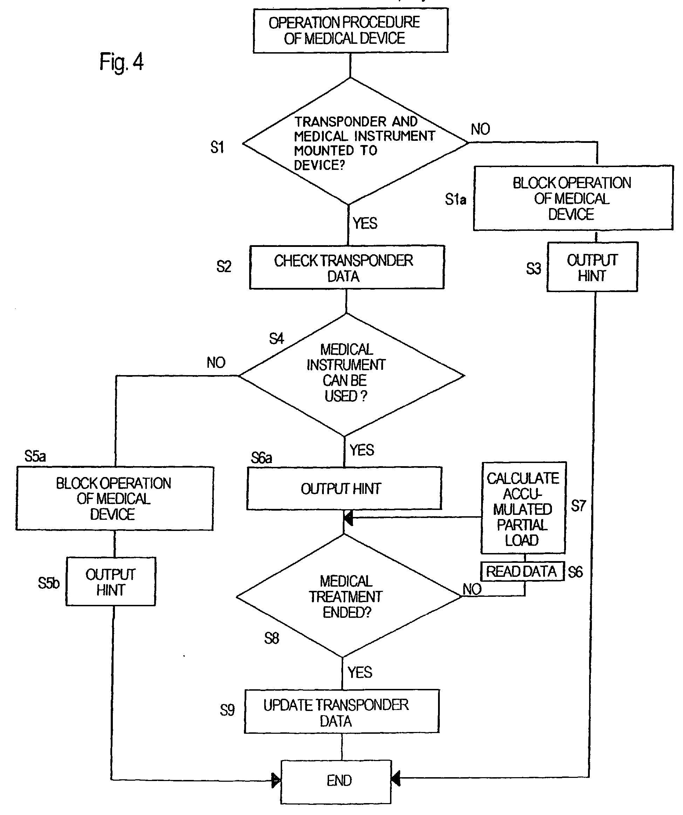

Below the functionality of the present invention will be described with respect to the flowchart of Fig 4.

As mentioned above, the medical instrument 2a with the transponder 5 detachably mounted thereon is stored in the magazine 6 in a respective hole 7 before the medical treatment begins which is in this case a dental treatment. The operator which is in this specific case the dentist takes out the corresponding medical instrument 2a with the transponder 5 mounted detachably thereon which is necessary for the corresponding dental treatment. Thereafter the operator separates the respective medical instrument 2a and the transponder 5 containing the specific information of the respective medical instrument 2a from each other. Then the medical instrument 2a is mounted to the receiving means 2 of the medical device 1, while the corresponding (associated) transponder 5 is fixed to the data exchange means 4 of the medical device 1. If the transponder is installed properly to the medical device 1 (S1), the medical device 1 is ready to carry out the corresponding dental treatment.

Before the dental treatment is performed, the data exchange means 4 and the transponder 5 exchange the specific information of the respective medical instrument 2a (S2). If the medical instrument 2a is in a state to perform the dental treatment, the medical device 1 can be operated by the operating means 3 so as to perform the corresponding treatment ("YES" in S4). Accordingly, a hint might be output (S6a) that the medical instrument 2a is in a condition suitable for carrying out the corresponding medical treatment.
If the data exchange between the medical device 1 and the transponder 5 results in that the medical instrument 2a is not suitable ("NO" in S4) due to wear or exceeding characteristic values as mentioned in the introductory part of the specification of the present invention, the medical instrument 2a will be blocked to operate (S5a) and thus the medical device 1 does not work.
Additionally, the operator may be warned by the operating display 3d of the operating means 3 which functions therefore as a warning means that the instrument is not suitable for a further dental treatment (S5b).

If the data exchange results in that the medical instrument 2a is suitable for a further medical treatment, the medical device 1 can be operated. There may be the possibility that the operating display 3d indicates that the condition of the corresponding medical instrument 2a is suitable to perform the dental treatment (S6a) as mentioned above.

While the dental treatment is carried out, data exchange is performed between the transponder 5 and the data exchange means 4 (S6, S7, S8, S9, S10). Thus, the transponder 5 receives new data from the data exchange means 4 and saves them. This is in more detail, that the data corresponding to the condition of the medical instrument 2a are read in (S6) which may be for example values like the rotational number, the torque or the rotational direction of the medical instrument 2a. This specific information of the corresponding medical instrument 2a is updated and accumulated to an accumulated partial load (S7) in order to achieve data representing the current condition of the medical instrument 2a. After the accumulated partial load is calculated, the transponder data are updated in a next step (S9) so that the respective transponder always has the latest information saved thereon. In more detail, the calculated accumulated partial load is added to the accumulated total load of the medical instrument 2a saved in the transponder 5 and, accordingly, a new updated accumulated total load is obtained which is then saved on the transponder 5 (S9). Consequently, the transponder 5 belonging to the respective medical instrument 2a contains the "up-to-date data" about the medical instrument 2a. Since the transponder always has the up-to-date data stored thereon, it is determined after step S9 if the medical instrument can still be used or is still in a condition suitable for the medical treatment (S10). If the medical instrument is in a condition ready for further usage ("YES" in step S10), the medical treatment can be carried out further. If the medical instrument is not in a condition suitable for the corresponding medical treatment ("NO" in step S10), the operation of the medical instrument will be blocked and a hint will be output (S11) which indicates that the medical instrument is not in a condition suitable for the medical treatment anymore. In this case the control routine of figure 4 ends.
This action can for example be performed by the electronic control unit which is part of the data exchange means 4.

The above described steps S6, S7, S8, S9, S10 will be carried out until the medical treatment has been finished, i.e., when the operator intends to finish the medical treatment.

If the medical treatment is completed, the operator removes the transponder 5 and the medical instrument 2a from the medical device 1, wherein the transponder 5 and the medical instrument 2a are fixed together again and put back to the magazine 6 for further activities which may be for example the cleaning of the medical instrument 2a after the medical treatment.

For the case that the medical instrument 2a is mounted to the receiving means 2 but the transponder 5 is not fixed correctly or not fixed at all to the data exchange means 4, the medical device is able to be operated ("NO" in S1) but a hint will output (S3) that the information of the medical instrument cannot be read in and, accordingly, that the information can also not be updated as described above. In this case the medical device 1 can be operated, especially in the case that a prior art medical instrument is used which does not have a transponder or the like for storing medical instrument specific information. Consequently, the operating display 3d of the medical device 1 may give the operator a hint to mount the respective transponder 5 belonging to the medical instrument 2a to the receiving means 2 (S3) if there is any but will not block the operation of the medical device 1.

As can be clearly seen from the above description of the medical device 1 with the medical instrument 2a and the corresponding transponder 5, no influence of any dental treatment affects the transponder 5 disadvantageously, since the transponder 5 and the medical instrument 2a are locally spaced apart during the dental treatment.

Furthermore, by the measures of protection, the respective medical instrument 2a and the respective transponder cannot not be confused, since the medical device 1 outputs a hint if the respective transponder 5 is not fixed to the data exchange means 4 correctly or at all. So a transponder 5 containing always the current or updated data with respect to the corresponding medical instrument 2a is assured.

The above-mentioned embodiment may be modified as described below:
In the above described embodiment the controlling and monitoring functions are carried out exclusively by the medical device 1 or the means contained in the medical device 1. However, these functions can also be carried out by the magazine 6. The difference to the above described embodiment would be the communication between the transponder 5 of the corresponding medical instrument 2a and the magazine 6. The corresponding medical instruments 2a would have to be checked out and checked in when taking from or placing to the magazine 6. As the case may be the magazine 6 would proceed the same steps as mentioned above and then determine based on the specific information of the corresponding medical instrument 2a if the latter could be used or not for the medical treatment. However, the transponder 5 has also to be updated during the medical treatment so that data exchange between the data exchange means 4 of the medical device 1 and the transponder 5 takes place. But the magazine 6 determines if the medical instrument 2a is suitable for a medical treatment.

A medical device comprises a medical instrument with a storage medium member, preferably a transponder, detachably fixed thereto. The transponder serves as data medium about specific information of the medical instrument. The medical device further comprises data exchange means for exchanging data with the transponder. When a medical treatment is to be performed, the medical instrument is placed to receiving means for receiving the medical instrument separated from the transponder, while the transponder is placed to the data exchange means which is placed locally apart or locally placed different from the receiving means so that disadvantageous influences of the medical treatment do not affect the transponder.

The represented embodiment and modification shall be only exemplary. With the embodiment and modification it is not intended to restrict the appended claims which represent the scope of protection of the present invention. The represented embodiment may be modified as long as the scope of protection of the appended claims is not left.

## Claims

1. A medical device comprising
a drill (2d) with a detachable storage medium member (5) formed thereon which serves as data medium about specific information of the drill (2d),
receiving means (2) for receiving the drill (2d) and data exchange means (4) for exchanging data with the storage medium member (5), **characterized in that**
the drill (2d) is placed to the receiving means (2) separated from the storage medium member (5), while the storage medium member (5) is placed to the data exchange means (4) which is locally placed apart from the receiving means (2).

2. The medical device according to claim 1, **characterized in that** the storage medium member (5) is preferably formed in the shape of a sleeve (5s) and comprises an engaging means (5e) for establishing an engagement between the storage medium member (5) and an engaging element of the data exchange means (4) and for establishing a connection able for data exchange.

3. The medical device according to claim 2, **characterized in that** the receiving means (2) is also able to drive, preferably to rotate the drill (2d) and drives the drill (2d) when the engagement between the storage medium member (5) and the data exchange means (4) is established.

4. The medical device according to claim 2 or 3, **characterized in that** the data exchange means (4) exchanges data with the storage medium member (5) when the engagement is established.

5. The medical device according to any preceding claim, **characterized in that** the specific information of the drill (2d) comprise information on the use of the drill (2d) required to calculate an accumulated total load on the drill (2d).

6. The medical device according to any preceding claim, **characterized in that** the specific information of the drill (2d) comprise preferably a position on a magazine in which the drill (2d) is to be stored when not used, a accumulated total and/or partial load , the number of sterilization cycles for the drill (2d) taking preferably into consideration especially parameters like the torque, the rotational direction and the rotational number of the drill (2d).

7. The medical device according to any preceding claim, **characterized in that** the drill (2d) is preferably a dentil drill.

8. The medical device according to any preceding claim, further comprising
operating means (3) for operating the receiving means (2) and
controlling means (3b) for controlling the operation of the receiving means (2).

9. The medical device according to claim 2 to 8, **characterized in that** the engagement between the storage medium member (5) and the data exchange means (4) is preferably established by a frictionally engaged connection and/or a form fitted connection, especially by one of a plug-and-socket-connection, a click-into-connection and a clamp-connection.

10. The medical device according to any preceding claim, **characterized in that** the data exchange means (4) includes
reading/writing means for reading the specific information from the storage medium member (5) and writing updated specific information to the storage medium member (5) which have been updated by calculating means for evaluating the specific information according to an operational state of the drill (2d), preferably by an electronic control unit.

11. The medical device according to any preceding claim, **characterized by** further comprising
warning means (3d) for warning an operator of the state of the drill (2d) if the specific information of the drill (2d) indicates a state not to proceed with the same.

## Patentansprüche

1. Medizinische Vorrichtung mit
einem Bohrer (2d) mit einem abnehmbaren Speichermediumelement (5), das daran ausgebildet ist, dass als Datenmedium hinsichtlich spezifischer Informationen des Bohrers (2d) dient,
einer Aufnahmeeinrichtung (2) zum Aufnehmen des Bohrers (2d) und
einer Datenaustauscheinrichtung (4) zum Austauschen von Daten mit dem Speichermediumelement (5), **dadurch gekennzeichnet, dass**
der Bohrer (2d) an der Aufnahmeeinrichtung (2) angeordnet ist, die von dem Speichermediumelement (5) getrennt ist, während das Speichermediumelement (5) an der Datenaustauscheinrichtung (4) angeordnet ist, die örtlich von der Aufnahmeeinrichtung (2) entfernt angeordnet ist.

2. Medizinische Vorrichtung gemäß Anspruch 1, **dadurch gekennzeichnet, dass** das Speichermediumelement (5) vorzugsweise in der Gestalt einer Manschette (5s) ausgebildet ist und eine Eingriffseinrichtung (5e) zum Bilden eines Eingriffs zwischen dem Speichermediumelement (5) und einem Eingriffselement der Datenaustauscheinrichtung (4) sowie zum Bilden einer Verbindung ausgebildet ist, die zum Datenaustausch fähig ist.

3. Medizinische Vorrichtung gemäß Anspruch 2, **dadurch gekennzeichnet, dass** die Aufnahmeinrichtung (2) ebenso fähig ist, den Bohrer (2d) anzutreiben, vorzugsweise zu drehen, und den Bohrer (2d) antreibt, wenn der Eingriff zwischen dem Speichermediumelement (5) und der Datenaustauscheinrichtung (4) hergestellt ist.

4. Medizinische Vorrichtung gemäß Anspruch 2 oder 3, **dadurch gekennzeichnet, dass** die Datenaustauscheinrichtung (4) Daten mit dem Speichermediumelement (5) austauscht, wenn der Eingriff hergestellt ist.

5. Medizinische Vorrichtung gemäß einem der vorhergehenden Ansprüche, **dadurch gekennzeichnet, dass** die spezifischen Informationen des Bohrers (2d) Informationen hinsichtlich der Verwendung des Bohrers (2d) umfassen, die zum Berechnen einer kumulierten Gesamtbelastung an dem Bohrer (2d) erforderlich sind.

6. Medizinische Vorrichtung gemäß einem der vorangehenden Ansprüche, **dadurch gekennzeichnet, dass** die spezifischen Informationen des Bohrers (2d) vorzugsweise eine Position an einem Magazin, in welchem der Bohrer (2d) aufzubewahren ist, wenn er nicht verwendet wird, eine kumulierte gesamte und/oder teilweise Belastung, die Anzahl der Sterilisationszyklen für den Bohrer (2d) vorzugsweise unter Berücksichtigung von speziellen Parametern wie Drehmoment, Drehrichtung und Drehzahl des Bohrers (2d) umfassen.

7. Medizinische Vorrichtung gemäß einem der vorangehenden Ansprüche, **dadurch gekennzeichnet, dass** der Bohrer (2d) vorzugsweise ein zahnmedizinischer Bohrer ist.

8. Medizinische Vorrichtung gemäß einem der vorangehenden Ansprüche, ferner mit
einer Betätigungseinrichtung (3) zum Betätigen der Aufnahmeeinrichtung (2) und
einer Steuereinrichtung (3b) zum Steuern des Betriebs der Aufnahmeeinrichtung (2).

9. Medizinische Vorrichtung gemäß Anspruch 2 bis 8, **dadurch gekennzeichnet, dass** der Eingriff zwischen dem Speichermediumelement (5) und der Datenaustauscheinrichtung (4) vorzugsweise durch eine Reibungseingriffsverbindung und/oder eine Formschlussverbindung, insbesondere durch eine Stecker-Buchse-Verbindung, eine Rastverbindung oder eine Klemmverbindung herbeigeführt wird.

10. Medizinische Vorrichtung gemäß einem der vorangehenden Ansprüche, **dadurch gekennzeichnet, dass** die Datenaustauscheinrichtung (4) umfasst
eine Lese/Schreibeinrichtung zum Lesen der spezifischen Informationen aus dem Speichermediumelement (5) und zum Schreiben von aktualisierten spezifischen Informationen in das Speichermediumelement (5), die durch die Berechnungseinrichtung aktualisiert wurden, zum Bewerten der spezifischen Informationen gemäß einem Betriebszustand des Bohrers (2d), vorzugsweise durch eine elektronische Steuereinheit.

11. Medizinische Vorrichtung gemäß einem der vorangehenden Ansprüche, ferner **gekennzeichnet durch**
eine Warneinrichtung (3d) zum Warnen eines Betreibers hinsichtlich des Zustands des Bohrers (2d), wenn die spezifischen Informationen des Bohrers (2d) einen Zustand anzeigen, der nicht fortgesetzt werden soll.

## Revendications

1. Dispositif médical comprenant
un foret (2d) avec un élément (5) de support de stockage amovible formé sur celui-ci qui sert de support de données pour des informations spécifiques du foret (2d),
un moyen (2) de réception destiné à recevoir le foret (2d) et
un moyen (4) d'échange de données destiné à échanger des données avec l'élément (5) de support de stockage, **caractérisé en ce que**
le foret (2d) est placé dans le moyen (2) de réception séparé de l'élément (5) de support de stockage, tandis que l'élément (5) de support de stockage est placé dans le moyen (4) d'échange de données qui est éloigné du moyen (2) de réception.

2. Dispositif médical selon la revendication 1, **caractérisé en ce que** l'élément (5) de support de stockage a de préférence la forme d'un manchon (5s) et comprend un moyen d'engagement (5e) destiné à établir un engagement entre l'élément (5) de support de stockage et un élément d'engagement du moyen (4) d'échange de données et à établir une connexion permettant l'échange de données.

3. Dispositif médical selon la revendication 2, **caractérisé en ce que** le moyen de réception (2) est également capable d'entraîner, de préférence de faire tourner le foret (2d) et entraîne le foret lorsque l'engagement entre l'élément (5) de support de stockage et le moyen (4) d'échange de données est établi.

4. Dispositif médical selon la revendication 2 ou 3, **caractérisé en ce que** le moyen (4) d'échange de données échange des données avec l'élément (5) de support de stockage lorsque l'engagement est établi.

5. Dispositif médical selon l'une des revendications précédentes, **caractérisé en ce que** les informations spécifiques du foret (2d) comprennent des informations concernant l'utilisation du foret (2d) nécessaires pour calculer une charge totale accumulée sur le foret (2d).

6. Dispositif médical selon l'une des revendications précédentes, **caractérisé en ce que** les informations spécifiques du foret (2d) comprennent de préférence une position sur un chargeur dans lequel le foret (2d) doit être stocké lorsqu'il n'est pas utilisé, une charge partielle et/ou totale accumulée, le nombre de cycles de stérilisation pour le foret (2d) en prenant de préférence en considération en particulier des paramètres tels que le couple, le sens de rotation et le nombre de rotations du foret (2d).

7. Dispositif médical selon l'une des revendications précédentes, **caractérisé en ce que** le foret (2d) est de préférence un foret dentaire.

8. Dispositif médical selon l'une des revendications précédentes, comprenant en outre
un moyen de manoeuvre (3) destiné à faire fonctionner le moyen (2) de réception et
un moyen (3b) de commande destiné à commander le fonctionnement du moyen (2) de réception.

9. Dispositif médical selon les revendications 2 à 8, **caractérisé en ce que** l'engagement entre l'élément (5) de support de stockage et le moyen (4) d'échange de données est établi de préférence par un raccordement engagé par friction et/ou un raccordement ajusté à la forme, en particulier par l'un d'un raccordement mâle et femelle, d'un raccordement à cliquet et d'un raccordement par serrage.

10. Dispositif médical selon l'une des revendications précédentes, **caractérisé en ce que** le moyen (4) d'échange de données comporte
un moyen de lecture/écriture destiné à lire les informations spécifiques de l'élément de support de stockage (5) et à écrire des informations spécifiques actualisées à l'élément de support de stockage (5) qui ont été mises à jour par un moyen de calcul destiné à évaluer les informations spécifiques selon un état de fonctionnement du foret (2d), de préférence par une unité de commande électronique.

11. Dispositif médical selon l'une des revendications précédentes, **caractérisé par** le fait de comprendre
un moyen d'avertissement (3d) destiné à avertir un opérateur de l'état du foret (2d) si les informations spécifiques du foret (2d) indiquent un état où il faut arrêter le fonctionnement du foret.
